# EUROPÄISCHE PATENTSCHRIFT

(11) **EP 0 881 490 B1**
(45) Veröffentlichungstag und Bekanntmachung des Hinweises auf die Patenterteilung: **10.09.2003**
(21) Anmeldenummer: 98109628.2
(22) Anmeldetag: 27.05.1998
(51) Int. Cl.: G01N 33/48, G01N 33/487, C12M 1/34

(54) **Messeinrichtung zum Messen von Eigenschaften einer lebenden Zelle**
Apparatus for measuring characteristics of a living cell
Appareil pour la mesure de caractéristiques d'une cellule vivante

(30) Priorität: 28.05.1997 DE 19722371; 19.07.1997 DE 19731078
(43) Veröffentlichungstag der Anmeldung: 02.12.1998
(73) Patentinhaber: Micronas GmbH, 79108 Freiburg (DE)
(72) Erfinder: Wolf, Bernhard, Prof. Dr., 79252 Stegen (DE); Sieben, Ulrich, Dr. Dipl.-Phys., 79276 Reute (DE)
(74) Vertreter: Sauer, Wolfgang

(56) Entgegenhaltungen:
- WO-A-95/31716
- DE-A- 4 013 588
- US-A- 5 278 048

## Beschreibung

Die Erfindung betrifft eine Meßeinrichtung zur Messung von physiologischen und/oder chemischen und/oder physikalischen Eigenschaften mindestens einer lebenden Zelle, die imobilisiert mit der Meßeinrichtung gekoppelt ist. Hierzu wird die Zelle veranlaßt, sich mit einer Trägereinrichtung ortsfest zu verbinden, die zur Messung der einzelnen Zellparameter eine Vielzahl von Sensoren aufweist. Das Material der Trägereinrichtung besteht dabei insbesondere aus einem Halbleitermaterial. Meßeinrichtungen mit derartigen Sensoren sind beispielsweise in WO 95/31716 (intern: C-DIT-1678) oder in DE 195 12117 A1 (intern: C-DIT-1680) beschrieben. Nachteilig bei den bekannten Anordnungen ist, daß die Beobachtung der lebenden Zellen vorwiegend über eine optische Mikroskopiereinrichtung erfolgt und die Meßeinrichtung daher einseitig offen ist oder mindestens über ein optisches Fenster verfügen muß. Dies erfordert eine relativ aufwendig herzustellende Meßstruktur, die teilweise aus durchsichtigem Material, insbesondere Glas oder optisch klaren Kunststoffen, besteht. Bei offenen Meßstrukturen ist zudem die horizontale Lage Voraussetzung, weil andernfalls Flüssigkeiten austreten können. Die Kombination mit den physiologischen Sensoren, die über elektronische Signale Eigenschaften der lebenden Zellen messen, und die in der Regel aus undurchsichtigen Halbleitermaterialien bestehen, wird dadurch deutlich erschwert.

In der bereits genannten Meßeinrichtung aus DE 195 12 117 A1 zur Messung und Untersuchung physiologischer Parameter an lebenden biologischen Zellen, insbesondere Zellverbänden, sind neben der Vielzahl von Sensoren zur Erfassung physiologischer Parameter auch optische Sensoren zur Erfassung von morphologischen Veränderungen der Zellen erwähnt, deren elektronische Ausgangssignale mittels geeigneter Schaltungseinrichtungen nach einer Analog-Digitalumsetzung einer externen Auswerteeinrichtung zugeführt sind. Als optische Sensoren werden Zeilen- oder Array-Anordnungen von CCD-Sensoren, insbesondere in Verbindung mit monolithisch integrierten Interdigitalkondensatoren angegeben.

In EP 0 751 393 A1 und WO 91/09 300 sind Meßverfahren und Meßanordnungen beschrieben, mit denen bei lebenden Zellen Fluoreszenzreaktionen erfaßt werden. Dabei reagieren die auf einer Trägereinrichtung befindlichen Zellen oder Zellverbände auf die Zugabe von chemischen oder biologischen Substanzen mit einer schwachen Lichtemission. Geeignete Lichtdetektoren, ev. unter Mithilfe von Lichtverstärkern, erfassen diese Lichtemission. Die Auswertung erfolgt über die Intensität, die Lichtwellenlänge, das Spektrum oder über die räumliche Verteilung der jeweiligen Fluoreszenzeffekte, die bei genügend hoher Auflösung auch Detailinformationen bezüglich einer einzigen Zelle liefern.

Es ist Aufgabe der Erfindung, den optischen Zugang zu lebenden Zellen auf einer Trägereinrichtung zu verbessern, um den aktuellen Zellzustand jederzeit leicht überprüfen zu können und dabei insbesondere eine gestaltverändernde und/oder biochemische Reaktion während des Meßvorganges zu erfassen.

Die Lösung dieser Aufgabe erfolgt entsprechend dem kennzeichnenden Merkmal des Anspruchs 1 dadurch, daß eine elektronische Steuereinrichtung mit den Optosensoren und einer aus einer Vielzahl von einzelnen Strahlungsquellen bestehenden Beleuchtungseinrichtung verkoppelt ist, um durch gezieltes Umschalten der Beleuchtungseinrichtung und Erfassen der jeweiligen Signale der Optosensoren in Verbindung mit einer externen Signalauswerteeinrichtung eine geometrische und/oder biochemische Information über die lebende Zelle zu erhalten.

Der Grundgedanke der Erfindung besteht darin, zur Erkennung der räumlichen Zellgeometrie und/oder der extrazellulären Produkte, beispielsweise Kalzium, die aus einzelnen Strahlungsquellen bestehende Beleuchtungseinrichtung und/oder die Optosensoren, von denen einige mit einer frequenzselektiven und/oder fluoreszierenden Schicht versehen sind, systematisch umzuschalten, um aus den unterschiedlichen Sensorsignalen durch geeignete Bildverarbeitungsverfahren die räumliche Zellgeometrie mindestens angenähert zu bestimmen und/oder eine Information über die extrazellulären Produkte zu erhalten. Die miniaturisierbaren elektrooptischen Sensoren befinden sich sehr nahe bei der Zelle und werden zusammen mit anderen Sensoren, die beispielsweise ebenfalls ionensensitiv oder stoffsensitiv sein können, auf einem gemeinsamen Halbleitersubstrat als Trägereinrichtung monolithisch integriert. Eine derartige Anordnung stellt unter anderem ein sensorgestüztes elektrooptisches Nahfeldmikroskop dar.

Wenn man als Trägereinrichtung für die Zellen und die Ausbildung der Sensoren ein monolithisch integrierbares Halbleitermaterial verwendet, läßt sich auch eine monolithisch integrierte Schaltung auf dem selben Substrat herstellen, wodurch in unmittelbarer Nähe des Meßobjektes eine Vorverarbeitung stattfinden kann. Es handelt sich somit um eine "intelligente" Sensoreinrichtung, die wesentlich mehr leistet, als rein passive Sensoren. Zumindest können die elektronischen Ausgangssignale der elektrooptischen Sensoren durch eine mitintegrierte Schaltung so aufbereitet werden, daß sie über Ausgangsschaltungen und Anschlußkontakte relativ problemlos nach außen geführt werden können. Beispielsweise kann die Vorverarbeitung aus der Digitalisierung der analogen Sensor- oder Meßsignale und ihrer Umwandlung in einen geeigneten Datenstrom bestehen. Darüberhinaus sind auch weitere Verarbeitungsschritte möglich, mit denen z.B. die Datenmenge reduziert werden kann oder die der externen Verarbeitung und Darstellung dienen. Damit ist es möglich, daß die verbleibende Auswertung der optischen und der anderen Signale und ihre Darstellung über einen Personal Computer (= PC) erfolgen kann. Die Steuerung der zugehörigen Einrichtungen auf dem Substrat erfolgt über Steuersignale aus einer Steuereinrichtung, die ebenfalls ganz oder teilweise auf dem Substrat ausgebildet sein kann oder extern angeschlossen wird.

Die Auswertung der optischen Signale über einen handelsüblichen Computer hat den weiteren Vorteil, daß über geeignete Programme eine weitgehende Automatisierung der Bildauswertung sowie eine Bildspeicherung möglich ist, so daß der Betrachter ganz andere Möglichkeiten hat als bei der einfachen Mikroskopbetrachtung. Über die Bildspeicherung wird z.B. eine Zeitrafferauswertung oder eine beliebig häufige Wiederholung bestimmter Bildsequenzen auf einfache Weise möglich. Bei genügender Dichte der elektrooptischen Sensoren dient der Bildschirm dem Betrachter als vollwertiger Mikroskopersatz. Dabei ist durch den variablen Betrachtungsabstand beim Bildschirm die Betrachtung selbst ermüdungsfreier als beim Mikroskop.

Die Beleuchtung der Zelle erfolgt über eine Beleuchtungseinrichtung, die optisch und mechanisch so mit den Optosensoren gekoppelt ist, daß ein Strahlungsfeld in Richtung der Optosensoren erzeugt wird, wobei der räumliche Abstand der Beleuchtungseinrichtung von den Optosensoren möglichst klein ist. Der Abstand muß dabei ausreichend bleiben, um die Zellen auf dem Substrat nicht zu behindern. Dabei kann es zweckmäßig sein, daß die Beleuchtungseinrichtung aus einer Vielzahl von punktförmigen Strahlungsquellen besteht, die mittels der Steuereinrichtung einzeln oder in Gruppen aktivierbar sind. Dies ermöglicht eine Auswertung der räumlichen Gestalt der Zelle, beispielsweise durch unterschiedliche Lichtintensitätszonen infolge der unterschiedlichen Lichtbrechung von Zelle und umgebendem Medium. Dies entspricht gleichsam den unterschiedlichen Schattenzonen bei einem undurchsichtigen Körper, der aus verschiedenen Richtungen beleuchtet wird. Eine ähnliche Wirkung hat es, wenn der Abstand zwischen der Beleuchtungseinrichtung und den Optosensoren über eine elektronisch steuerbare Stelleinrichtung definiert veränderbar ist.

Die Anordnung der punktförmigen Strahlungsquellen, die z.B. aus gebündelten Lichtleiterfasem oder aus miniaturisierten LED (= Light Emitting Diode) bestehen oder auf andere Weise realisiert sind, ist zweckmäßigerweise zeilen- oder feldförmig. Um bestimmte Strukturmerkmale der Zelle zu erfassen, kann es vorteilhaft sein, daß die Strahlungsquellen in der Frequenz durchstimmbar sind oder daß Strahlungsquellen unterschiedlicher Frequenz vorhanden sind. Dem entspricht, daß auf der Sensorseite nicht nur unterschiedliche Sensoren für die physiologischen und/oder chemischen und/oder physikalischen Messungen zur Verfügung stehen, sondern gegebenenfalls auch unterschiedliche oder durchstimmbare oder umschaltbare Sensoren für die optische Auswertung. Durch eine geeignete optische Zusatzschicht auf den optischen Sensoren können diese für unterschiedliche Wellenlängen sensibilisiert werden. Eine weitere Möglichkeit stellt die selektive Beschichtung einzelner Optosensoren mit Fluoreszenzfarbstoffen dar, die auf verschiedene bio/chemische Stoffe selektiv mit Fluoreszenz reagieren. Auf diese Weise können beispielsweise Kalzium- und/oder Natriumionen und/oder Sauerstoff und/oder anderes nachgewiesen werden. Dies ermöglicht eine gleichzeitige chemische und geometrische Information über die Zelle.

Die Erfindung und vorteilhafte Ausgestaltung werden nun anhand der Figuren der Zeichnung näher erläutert:
Fig. 1 zeigt schematisch eine Meßeinrichtung nach der Erfindung, die mit einem Personal Computer gekoppelt ist,
Fig. 2 zeigt schematisch in Aufsicht einen Ausschnitt eines Sensorfeldes mit einer Vielzahl von optischen und weiteren Sensoren und
Fig. 3 zeigt schematisch in Seitenansicht eine Zelle auf dem Sensorfeld mit verschiedenen Lichtintensitätszonen.

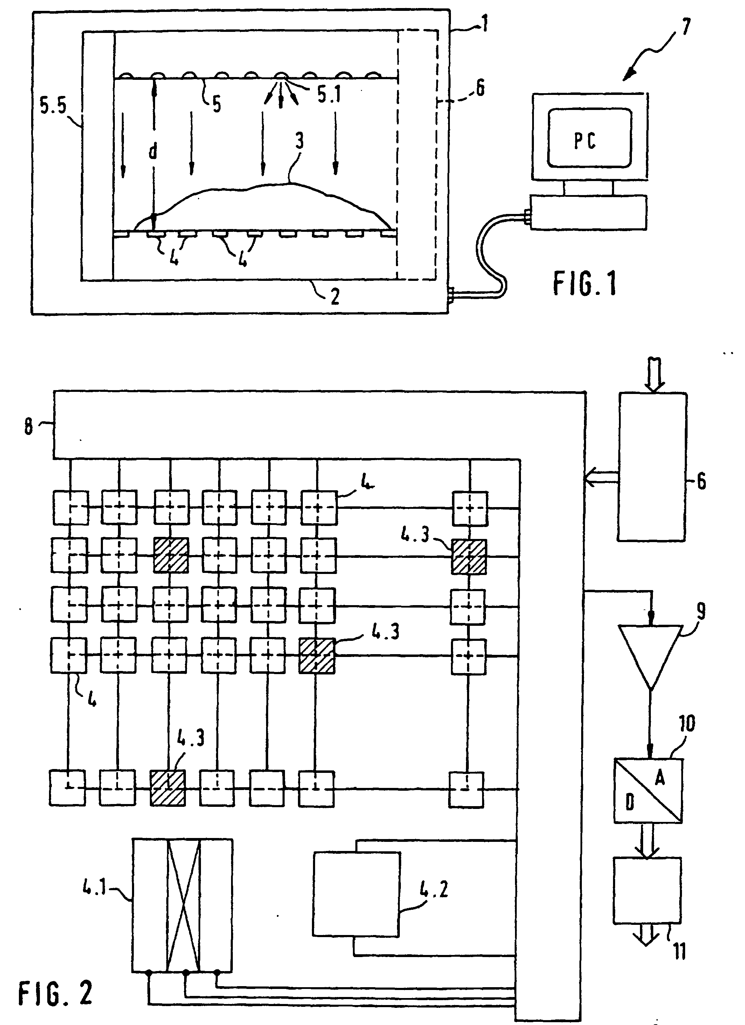

In Fig. 1 umfaßt der Block 1 diejenigen Teile der Meßeinrichtung, die auch räumlich zusammengehören. Das ist eine Trägereinrichtung 2 für das Meßobjekt 3, das beispielsweise eine lebende Zelle sein kann, und die Sensoren 4. In einem räumlichen Abstand d von Trägereinrichtung 2 befindet sich eine Beleuchtungseinrichtung 5, die aus einzelnen Beleuchtungsquellen 5.1 besteht, die flächenförmig oberhalb des Meßobjektes angeordnet sind. Eine homogene Beleuchtungsquelle ist auch denkbar, jedoch sind einzeln ansteuerbare Beleuchtungsquellen von Vorteil wie später noch ausführlich gezeigt wird. In der Regel enthält der Block 1 auch eine elektronische Steuereinrichtung 6. Diese muß jedoch nicht zwingend im Block 1 angeordnet sein, denn sie kann sich auch völlig außerhalb der Meßeinrichtung 1 befinden oder teils außerhalb und teils innerhalb. Auch können einige Steuerfunktionen von dem angeschlossenen PC übernommen werden. Besonders vorteilhaft ist es, wenn die Trägereinrichtung 2, die Sensoren 4 und die Steuereinrichtung 6 eine bauliche Einheit bilden, weil dann mindestens ein Teil der Steuereinrichtung 6 direkt Zugang zu den Sensoren 4 hat, wodurch die Anzahl nach außen zu führenden elektrischen Signal- und Steuerleitungen sehr reduziert werden kann. Als Trägermaterial 2 eignen sich insbesondere Halbleitermaterialien, beispielsweise Silizium, weil sie einmal eine gute Verträglichkeit mit lebenden Zellen aufweisen, gegebenenfalls unter Verwendung geeigneter Passivierungsmaßnahmen, und weil sie zum anderen bekannte Herstellungsverfahren für monolithisch integrierte Schaltungen zulassen. Da als Material für die Trägereinrichtung 2 insbesondere derartige Halbleitermaterialien angesprochen sind, wird vereinfachend auch der aus der Halbleitertechnik vertraute Begriff "Substrat" für die Trägereinrichtung 2 verwendet. Der Abstand der Beleuchtungseinrichtung 5 von der Trägereinrichtung 2 kann mittels einer elektronisch steuerbaren Stelleinrichtung 5.5 verändert werden, wobei auch die Ebenen gegebenenfalls gegeneinander neigbar sind, um auch leicht geneigte Strukturen optimal darstellen zu können.

Die Immobilisierung und Ernährung der einen lebenden Zelle 3 oder der mehreren lebenden Zellen auf der Trägereinrichtung 2 erfolgt durch Verfahren und Maßnahmen, die in den oben angegebenen Patentanmeldungen ausführlich beschrieben sind. Die hierzu erforderlichen Anschlüsse oder Einrichtungen sind in Fig. 1 nicht dargestellt. Der eigentliche Meßbereich der Meßeinrichtung 1 ist im wesentlichen auf die Größe der lebenden Zelle 3 abgestimmt. Die möglichst nahe beieinanderliegenden optischen Sensoren 4 sind mindestens eine Größenordnung kleiner als die Zelle, sonst wird die optische Auflösung zu schlecht. Bei der zunehmenden Miniaturisierung der Sensortechnik und den entsprechenden Halbleitertechnologien ist diese Bedingung leicht zu erfüllen. Es ist hierbei sogar denkbar, daß durch die elektrooptische Nahfeldmikroskopie nach der Erfindung eine Auflösung erreicht wird, die unterhalb der Lichtwellenlänge liegt. Als Beleuchtungseinrichtung dienen z.B. miniaturisierte Leuchtdioden 5.1 (= LED), die zeilenweise oder als Feld angeordnet sind. Über eine sequentielle Ansteuerung der einzelnen Leuchtdioden und die sequentielle Auswertung der zugehörigen Sensorsignale kann eine Auswertung nach dem Tomographieprinzip erfolgen, sodaß sich ein dreidimensionales Bild der Zelle 3 darstellen läßt.

Der besseren Übersicht wegen sind in Fig. nur elektrooptische Sensoren 4 dargestellt. Eine Bestimmung möglichst vieler physiologischer und/oder chemischer und/oder physikalischer Parameter erfordert entsprechende ionensensitive oder stoffsensitive Sensoren auf der Trägereinrichtung 2 oder in deren Nachbarschaft, vgl. in Fig. 2 beispielsweise die weiteren Sensoren 4.1, 4.2. Diese Sensoren sind beispielsweise in der Art von Feldeffekttransistoren mit und ohne Gate-Anschluß ausgebildet. Die Steuerung des Gates und damit des Stromes erfolgt im wesentlichen durch die Reaktion der darüberliegenden Zelle auf unterschiedliche Umgebungszustände. Diese feldeffektähnlichen Sensoren 4.1, 4.2 sind in Fig. 2 deutlich größer als die optischen Sensoren 4 dargestellt. Sie umfassen beispielsweise jeweils eine etwa quadratische Fläche von 15 Mikrometer Seitenlänge auf dem Substrat. Diese Sensoren 4.1, 4.2 können jedoch bei einer weiteren Miniaturisierung ebenfalls so verkleinert werden, daß sie leicht in das optische Array einfügbar sind, vgl. dort z.B. die Sensoren 4.3.

Mit den Sensoren 4.1, 4.2, die entweder im Feld der elektrooptischen Sensoren 4 eingebettet oder zu diesem unmittelbar benachbart sind, lassen sich elektronische Signale abgreifen, die eine Aussage über den jeweiligen chemisch und/oder physiologischen Zustand des Meßobjektes oder der biologischen Struktur zulassen. Die Auswertung dieser charakterisierenden Signale in Verbindung mit der elektrooptischen Auswertung ergibt schnelle und eindeutige Meßergebnisse. Über die Änderung der Umgebung der Zelle 3, z.B. durch Zugabe von physiologisch wirksamen Substanzen, die in Wechselwirkung mit dem Stoffwechsel der Zelle treten, lassen sich auf diese Weise rasch Erkenntnisse gewinnen, wie die Zelle 3 oder das Meßobjekt auf die zugegebenen Substanzen reagiert.

In Fig. 2 ist schematisch eine Aufsicht auf das Feld der elektrooptischen Sensoren 4 dargestellt. Diese rasterförmige Anordnung erlaubt eine gezielte Einzelabtastung der Sensoren. Nacheinander werden so alle Sensoren zeilenweise abgefragt und als entsprechende Bildpunkte auf dem Bildschirm des PC's dargestellt. Die Abtastung der einzelnen Sensoren 4 wird von der Schalteinrichtung 8 gesteuert, die vorteilhafterweise auf dem Substrat mitintegriert ist. Hierzu steht die Schalteinrichtung 8 über eine Busverbindung mit der Steuereinrichtung 6 in Verbindung, die ihrerseits Steuersignale von dem angeschlossenen PC bekommt. Die Steuereinrichtung 6 dient dabei auch als Interface-Schaltung, um die Daten des PC in Steuersignale für die Schalteinrichtung 8 umzusetzen, die letztendlich die Ansteuersignale für die einzelnen Sensoren 4, 4.1, 4.2, 4.3 bildet, und auch deren Antwortsignale empfängt. Die Signale der einzelnen Sensoren sind analoge Spannungs- oder Stromwerte, die vor der weiteren Verarbeitung in der Regel zu digitalisieren sind. Dies erfolgt zweckmäßigerweise im Zusammenhang mit der Schalteinrichtung 8 in unmittelbarer Nähe der Sensoren, weil dadurch mögliche Signalverfälschungen weitgehend unterdrückt werden können. Mittels eines Verstärkers 9 werden die schwachen Meßsignale verstärkt, bevor sie in einem Analog-Digitalumsetzer 10 digitalisiert werden. Diese Daten werden dann über eine Ausgangsschaltung 11 parallel, seriell oder in gemischter Form nach außen geführt. Bei Verwendung eines Halbleitermaterials als Trägereinrichtung 2 kann sowohl die Steuereinrichtung 6 als auch die Eingangsschaltung 11 auf dem Substrat mitintegriert werden. Gegebenenfalls können auch beide Schaltungen 6, 11 mit einer gemeinsamen, bidirektionalen Ein/Ausgangsschaltung zusammenarbeiten, wodurch sich die Anzahl nach außen zu führenden Leitungen weiter verringert. Wenn die Anzahl der Sensoren eine ausreichende optische Auflösung ermöglichen soll, dann ist eine intelligente Vorverarbeitung der riesigen Datenmenge auf der Trägereinrichtung 2 erforderlich, weil die Vielzahl der auswertenden Sensorsignale schon aus mechanischen Gründen wegen der Enge gar nicht von der Trägereinrichtung 2 abgreifbar wären.

Im Array der optischen Sensoren 4 von Fig. 2 sind einige Sensoren 4.3 durch Schrägschraffur dargestellt. Es handelt sich hierbei entweder um modifizierte optische Sensoren 4, die z.B. für eine andere Wellenlänge optimiert sind oder um miniaturisierte Sensoren im Sinne der Sensoren 4.1, 4.2, die einen chemischen und/oder physiologischen und/oder physikalischen Parameter der Zelle 3 bestimmen.

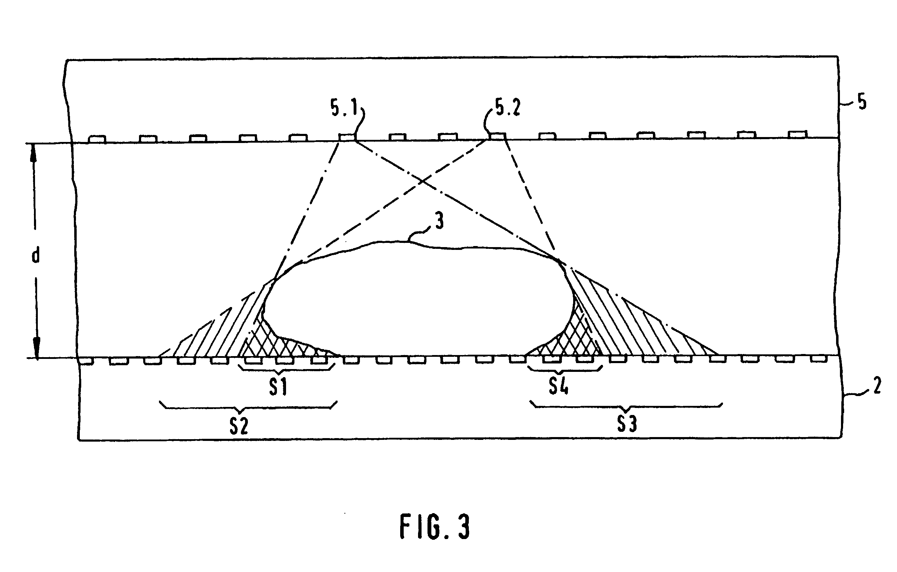

In Fig. 3 ist schematisch in Seitenansicht eine Zelle 3 auf der Trägereinrichtung 2 dargestellt. Hierbei schmiegt sich die Zelle nicht so gut an die Oberfläche der Trägereinrichtung an wie in Fig. 1. Bei der Beleuchtung durch einzelne Strahlungsquellen 5.1, 5.2 innerhalb der Beleuchtungseinrichtung 5 entstehen im Randbereich der Zelle 3 unterschiedliche Lichtintensitätszonen S1, S2, S3, S4, die sich teilweise überlappen, z.B. die Zonen S1, S2 und die Zonen S3, S4. Die unterschiedliche Ausbildung der Lichtintensitätszonen führt bei den optischen Sensoren 4 zu unterschiedlichen Umrißkonturen, die in der Auswerteeinrichtung gespeichert und miteinander verarbeitet werden. Es lassen sich dabei tomographieähnliche Darstellungen gewinnen, die eine räumliche Aussage über die Zellform und insbesondere mögliche Änderungen der Form zulassen. Eine wichtige Aussage über das momentane Befinden der Zelle 3 ist die Information, in wieweit sich die Zelle von ihrer Unterlage löst. Wenn das Umfeld einschließlich der Trägeroberfläche für epithelartige Zellen positiv ist, dann sind sie bestrebt, sich möglichst dicht an die Trägeroberfläche anzuschmiegen. Wenn das Umfeld jedoch ungünstig oder gar zellfeindlich wird, dann versuchen die Zellen zunächst eine kleinere Oberfläche anzunehmen. Sie ziehen sich hierfür zusammen bis sie sich im Extremfall von der Unterlage ablösen und schließlich sterben. Das Zusammenziehen erfolgt zuerst im Randbereich und ist daher durch die beschriebene Änderung der Lichtintensitätszonen S1, bis S4 leicht festzustellen.

Auch eine Änderung des Abstandes d der punktförmigen Strahlungsquelle bewirkt ebenfalls eine Änderung der Lichtintensitätszone, die als räumliche Information ausgewertet werden kann. Die Änderung der Lichtintensitätszone ist auch beobachtbar, wenn statt der punktförmigen Strahlungsquellen zeilenförmige Gruppen von Strahlungsquellen umgeschaltet werden.

Die Überwachung der Zellform auf diese Weise ist sehr effektiv, denn die Wirkung der zu untersuchenden Substanz auf den Zellzustand kann bereits im Anfang der Zugabe oder bei schwachen Konzentrationen überprüft werden und man muß nicht abwarten, bis eine irreversible Zellschädigung oder gar der Zelltod eingetreten ist. In diesem Anfangsbereich reagiert die Zelle aktiv auf die feindlichen Substanzen und der Zustand ist reversibel, wenn das die Zelle umgebende Medium wieder zellfreundlicher wird. Man kommt somit sehr rasch zu den zutreffenden Aussagen über die Wirkung der zu untersuchenden Substanzen auf die Zelle. Durch den schonenden Umgang steht somit die Meßanordnung 1 mit der lebenden Zelle 3 für weitere Untersuchungen zur Verfügung. Dies ermöglicht eine Vielzahl von Einzeluntersuchungen, die ohne die Meßeinrichtung nach der Erfindung sehr aufwendig wären. Die optische Überwachung der Zellform in Verbindung mit den Aussagen der anderen Sensoren stellt somit eine äußerst leistungsfähige Meßeinrichtung dar.

## Patentansprüche

1. Meßeinrichtung (1) zur Messung von physiologischen und/oder chemischen und/oder physikalischen Eigenschaften mindestens einer lebenden Zelle (3), die immobilisiert mit der Meßeinrichtung gekoppelt ist, wobei die Meßeinrichtung eine Vielzahl von Sensoren (4; 4.1, 4.2, 4.3) auf einer Trägereinrichtung (2) enthält, die insbesondere aus einem Halbleitermaterial besteht, und die Vielzahl von Sensoren Optosensoren (4) enthalten, wobei die Optosensoren (4) feldförmig angeordnet sind, **dadurch gekennzeichnet, daß** eine elektronische Steuereinrichtung (6) mit den Optosensoren (4) und einer Vielzahl von einzelnen Strahlungsquellen (5.1; 5.1, 5.2) bestehenden Beleuchtungseinrichtung (5) verkoppelt ist, um durch gezieltes Umschalten der Beleuchtungseinrichtung (5) und gezieltes Abfragen der Optosensoren (4) in Verbindung mit einer externen Signalauswerteeinrichtung (7) eine geometrische und/oder biochemische Information über die lebende Zelle (3) zu erhalten.

2. Meßeinrichtung (1) nach Anspruch 1, **dadurch gekennzeichnet, daß** die Ausgangssignale der Optosensoren (4) mittels eines auf dem Substrat ausgebildeten Analog/Digital-Umsetzers (10) digitalisiert sind.

3. Meßeinrichtung (1) nach Anspruch 2, **dadurch gekennzeichnet, daß** die Ausgangssignale des Analog/Digital-Umsetzers (10) einem Rechner, insbesondere einem Personal Computer (7) als externer Auswerte- und Wiedergabeeinrichtung zugeführt sind.

4. Meßeinrichtung (1) nach einem der Ansprüche 1 bis 3, **dadurch gekennzeichnet, daß** die Beleuchtungseinrichtung (5) ein Strahlungsfeld in Richtung der Optosensoren (4) erzeugt, wobei der räumliche Abstand d der Beleuchtungseinrichtung von den Optosensoren mindestens so groß ist, daß die mindestens eine lebende Zelle (3) auf der Trägereinrichtung (2) nicht behindert ist.

5. Meßeinrichtung (1) nach Anspruch 4, **dadurch gekennzeichnet, daß** der räumliche Abstand d und/oder die relative Ausrichtung der Beleuchtungseinrichtung (5) zu den Optosensoren (4) durch eine elektronisch gesteuerte Stelleinrichtung (5.5) einstellbar ist.

6. Meßeinrichtung (1) nach Anspruch 4, **dadurch gekennzeichnet, daß** die Beleuchtungseinrichtung (5) aus einer Vielzahl von unabhängig ansteuerbaren Strahlungsquellen (5.1; 5.1, 5.2) besteht, die mittels der Steuereinrichtung (6) einzeln oder in Gruppen aktivierbar sind.

7. Meßeinrichtung (1) nach einem der Ansrpüche 1 bis 6, **dadurch gekennzeichnet, daß** die Optosensoren (4) und/oder die Beleuchtungseinrichtung (5) bezüglich des Wellenlängenbereichs unterschiedlich oder durchstimmbar oder umschaltbar sind.

8. Meßeinrichtung (1) nach Anspruch 7, **dadurch gekennzeichnet, daß** mindestens ein Teil der Optosensoren (4) mit einer Fluoreszenzfarbstoffschicht versehen ist.

9. Meßeinrichtung (1) nach einem der Ansprüche 1 bis 8, **dadurch gekennzeichnet, daß** die Auswerteeinrichtung (7) Strukturgrößen der Zellgeometrie über Bildauswerteverfahren automatisch bestimmt und ausgewertet.

10. Meßeinrichtung (1) nach Anspruch 9, **dadurch gekennzeichnet, daß** die Auswerteeinrichtung (7) die Änderungen der Strukturgrößen der Zellgeometrie in einer Zeitrafferdarstellung wiedergibt.

## Claims

1. A measuring apparatus (1) for measuring physiological and/or chemical and/or physical properties of at least one living cell (3) which is immobilized and coupled with the measuring apparatus, the measuring apparatus comprising a plurality of sensors (4; 4.1, 4.2, 4.3) on a support (2), particularly on a support made of semiconductor material, and the plurality of sensors comprising optical sensors (4) arranged in the form of a matrix array, **characterized in that** an electronic controller (6) is coupled to the optical sensors (4) and to an illuminant (5) consisting of a plurality of individual radiation sources (5.1; 5.1, 5.2) for obtaining geometric and/or biochemical information about the living cell (3) by selective switching of the illuminant (5) and selective interrogation of the optical sensors (4) in conjunction with an external signal-analyzing facility (7).

2. A measuring apparatus (1) as claimed in claim 1, **characterized in that** the output signals from the optical sensors (4) are digitized by means of an analog-to-digital converter (10) implemented on the substrate.

3. A measuring apparatus (1) as claimed in claim 2, **characterized in that** the output signals from the analog-to-digital converter (10) are fed to a computer used as an external analyzing and reproduction facility, particularly to a personal computer (7).

4. A measuring apparatus (1) as claimed in any one of claims 1 to 3, **characterized in that** the illuminant (5) produces a radiation field in the direction of the optical sensors (4), with the distance d of the illuminant from the optical sensors being at least such that the at least one living cell (3) on the support (2) is not hindered.

5. A measuring apparatus (1) as claimed in claim 4, **characterized in that** the distance d and/or the orientation of the illuminant (5) relative to the optical sensors (4) is adjustable by an electronically controlled actuator (5.5).

6. A measuring apparatus (1) as claimed in claim 4, **characterized in that** the illuminant (5) consists of a plurality of independently activatable radiation sources (5.1; 5.1, 5.2) which are activatable separately or in groups by means of the controller (6).

7. A measuring apparatus (1) as claimed in claim 1, **characterized in that** the optical sensors (4) and/or the illuminant (5) differ in wavelength range or are continuously variable over their wavelength range or that their wavelength range is switch-selectable.

8. A measuring apparatus (1) as claimed in claim 7, **characterized in that** at least part of the optical sensors (4) are provided with a coating of a fluorescent dye.

9. A measuring apparatus (1) as claimed in any one of claims 1 to 8, **characterized in that** the analyzing facility (7) automatically determines and analyzes structure parameters of the cell geometry using image analysis techniques.

10. A measuring apparatus (1) as claimed in claim 9, **characterized in that** the analyzing facility (7) reproduces the changes in the structure parameters of the cell geometry in fast motion.

## Revendications

1. Dispositif de mesure (1) pour la mesure de propriétés physiologiques et/ou chimiques et/ou physiques d'au moins une cellule vivante (3) qui est reliée au dispositif de mesure (1) en étant immobilisée, le dispositif de mesure (1) contenant une pluralité de capteurs (4 ; 4.1, 4.2, 4.3) qui sont disposés sur dispositif support (2), qui est constitué en particulier de matériau semi-conducteur, la pluralité de capteurs comprenant des capteurs optiques (4) qui sont disposés selon une configuration de champ, **caractérisé en ce qu'**un dispositif de commande électronique (6) est relié aux capteurs optiques (4) et à un dispositif d'illumination (5) qui est constitué d'une pluralité de sources de rayonnement séparées (5.1 ; 5.1, 5.2) pour obtenir des informations géométriques et/ou biochimiques concernant la cellule vivante (3) au moyen d'une commutation appropriée du dispositif d'illumination (5) et d'une interrogation appropriée des capteurs optiques (4) en liaison avec un dispositif externe de traitement de signaux (7).

2. Dispositif de mesure (1) selon la revendication 1, **caractérisé en ce que** les signaux de sortie des capteurs optiques (4) sont numérisés au moyen d'un convertisseur analogique-numérique (10) réalisé sur le substrat.

3. Dispositif de mesure (1) selon la revendication 2, **caractérisé en ce que** les signaux de sortie du convertisseur analogique-numérique (10) sont envoyés à un calculateur,, en particulier un ordinateur personnel (7), qui fonctionne en tant que dispositif d'évaluation et de reproduction.

4. Dispositif de mesure (1) selon l'une quelconque des revendications 1 à 3, **caractérisé en ce que** le dispositif d'illumination (5) produit un champ de rayonnement dans la direction des capteurs optiques (4), la distance spatiale, d, entre le dispositif d'illumination (5) et les capteurs optiques (4) étant au moins suffisamment grande pour que l'au moins une cellule vivante (3) ne soit pas gênée quand elle se trouve sur le dispositif support (2).

5. Dispositif de mesure (1) selon la revendication 4, **caractérisé en ce que** l'on peut régler la distance spatiale, d, et/ou l'orientation relative du dispositif d'illumination (5) par rapport aux capteurs optiques (4) au moyen d'un dispositif de réglage (5.5) qui, est commandé de manière électronique.

6. Dispositif de mesure (1) selon la revendication 4, **caractérisé en ce que** le dispositif d'illumination (5) est constitué d'une pluralité de sources de rayonnement (5.1 ; 5.1, 5.2) qui sont commandées de manière indépendante et qui peuvent être activées séparément ou par groupes par le dispositif de commande électronique (6).

7. Dispositif de mesure (1) selon l'une quelconque des revendications 1 à 6, **caractérisé en ce que** les capteurs optiques (4) et/ou le dispositif d'illumination (5) peuvent être réglés ou commutés de manière différente en ce qui concerne la gamme des longueurs d'onde.

8. Dispositif de mesure (1) selon la revendication 7, **caractérisé en ce qu'**au moins une partie des capteurs optiques (4) est munie d'une couche de matière colorée fluorescente.

9. Dispositif de mesure (1) selon l'une quelconque des revendications 1 à 8, **caractérisé en ce que** le dispositif d'évaluation (7) détermine et évalue, de manière automatique, des grandeurs de structure de la géométrie de la cellule par l'intermédiaire d'un procédé d'exploitation d'images.

10. Dispositif de mesure (1) selon la revendication 9, **caractérisé en ce que** le dispositif d'évaluation (7) restitue les modifications des grandeurs de structure par une représentation en accéléré.
